# EUROPÄISCHE PATENTSCHRIFT

(11) **EP 0 336 353 B1**
(45) Veröffentlichungstag und Bekanntmachung des Hinweises auf die Patenterteilung: **21.08.1996**
(21) Anmeldenummer: 89105858.8
(22) Anmeldetag: 04.04.1989
(51) Int. Cl.: C12Q 1/56

(54) **Verfahren zur kinetischen Bestimmung von Faktor XIII**
Method for the kinetic determination of Factor XIII
Procédé pour la détermination cinetique du facteur XIII

(30) Priorität: 07.04.1988 DE 3811647
(43) Veröffentlichungstag der Anmeldung: 11.10.1989
(73) Patentinhaber: BEHRINGWERKE Aktiengesellschaft, 35001 Marburg (DE)
(72) Erfinder: Stüber, Werner, Dr., D-3551 Lahntal (DE)

(56) Entgegenhaltungen:
- EP-A- 0 121 995
- EP-A- 0 314 023
- CLINICAL CHEMISTRY, Band 31, Nr. 1, 1985, Winston, NC (US); L. MUSZBEK et al., Seiten 35-40
- ANALYTICAL BIOCHEMISTRY, Band 144, 1985, New York, NY (US); C.C. MIRAGLIA et al., Seiten 165-171
- JOURNAL OF BIOLOGICAL CHEMISTRY, Band 255, Nr. 2, 25 Januar 1980, Baltimore, MD (US); J.J. GORMAN et al., Seiten 419-427
- JOURNAL OF BIOLOGICAL CHEMISTRY, Band 259, Nr. 14, 25 Juli 1984, Baltimore, MD (US); J.J. GORMAN et al., Seiten 9007-9010
- CLINICAL CHEMISTRY, Band 31, Nr. 12, Dezember 1985, Winston, NC (US); L. FESUS et al., Seiten 2044-2045
- DATABASE WPIL/DERWENT; Derwent Publications Ltd.; Nr. 86-255894
- C. DEBER et al. (Eds.): "Peptides: Structure And Function. Proceedings Of The American Peptide Symposium, 9th, TORONTO" 1985, Pierce Chemical Comp. ROCKFORD US, Seiten 363-366

## Beschreibung

Die Erfindung betrifft ein Verfahren und eine Verpackung enthaltend Mittel zur qualitativen und quantitativen Messung des Blutgerinnungsfaktors XIII.

Faktor XIII ist eine Transglutaminase, die im Plasma als Proenzym zirkuliert und bei Bedarf durch Thrombin aktiviert wird. Daneben zeigen auch Thrombozyten und Plazenten Faktor XIII-Aktivität. Die biochemische Wirkung des Faktors XIII liegt in der Vernetzung von über Wasserstoffbrücken assoziierten Fibrinmonomeren, wodurch ein unlösliches Fibringerinnsel entsteht. Neben der Fibrin stabilisierenden Wirkung kommt dem Faktor XIII große Bedeutung bei der Wundheilung zu, indem die in das Gerinnsel einwachsenden Fibroblasten durch Faktor XIII beschleunigt ausgebildet werden.

Aufgrund der klinischen Relevanz von Faktor XIII kommt der Bestimmung dieses Gerinnungsfaktors eine hohe Bedeutung zu. Im wesentlichen werden zur quantitativen Messung die folgenden Methoden benutzt: Einbau eines radioaktiv markierten Substrats in ein Protein, z.B. Casein, Bestimmung der Löslichkeit/Unlöslichkeit eines Gerinnsels; Elektroimmunoassays sowie die Bestimmung des Ammoniakgehaltes während der Clotbildung. Diese Nachweismethoden sind mit dem Nachteil behaftet, daß sie zeitaufwendig oder nicht einfach durchführbar sind. Zielsetzung dieser Erfindung war deshalb, Verfahren und eine Verpackung enthaltend Mittel zu einer einfacheren und schnelleren F XIII- Bestimmung bereitzustellen.

Aus Analytical Biochemistry 144, 165-171 (1985) ist bekannt, F XIII in Plasma, aus dem das Fibrinogen nicht entfernt wurde, unter Zusatz von Glycyl-Prolyl-Arginyl-Prolin zu- bestimmen, wobei "Hammersten"-Casein als Substrat dient. Als Nachweismethode wurde der (³H) Putrescineinbau gewählt.

Aus Clinical Chemistry 31, 35 - 40 (1985), ist bekannt, die F XIII-katalysierte Ammoniakentwicklung mit dem Substrat Kasein/Ethylamin und dem Ammoniakreagens NADH/GLDH/ Ketogluterat zu bestimmen.

In Festus et al.(Clin.Chem. 31(12),1985, S.2044-2045),Gorman et al.(J.Biol.Chem.255(2),1980,S.419-427),Gorman et al.(J.Biol.Chem.259(14), 1984,S.9007-9010), EP-A 0 121 995 und Gorman et al.(in Peptides:Structure and Function. Proceedings of the American Peptide Symposium, 9th, Toronto (Eds.Deber et al.),1985, S.363-366) wird die Verwendung von glutaminhaltigen Peptiden , aus denen durch Faktor XIII Ammoniak abgespalten werden kann, beschrieben. In dem älteren Recht EP-A 0 314 023 wird die generelle Verwendung- bestimmter Peptidsubstrate beschrieben.

Wir haben überraschenderweise gefunden, daß sich die Bestimmung von F XIII in Plasma verbessern läßt, indem ein glutaminhaltiges Peptid. als F XIII-Substrat in Kombination mit einem Fibrinpolymerisationshemmer eingesetzt wird und der F XIII über das gebildete Ammoniak mittels einer nachfolgenden NADH-abhängigen Reaktion bestimmt wird.

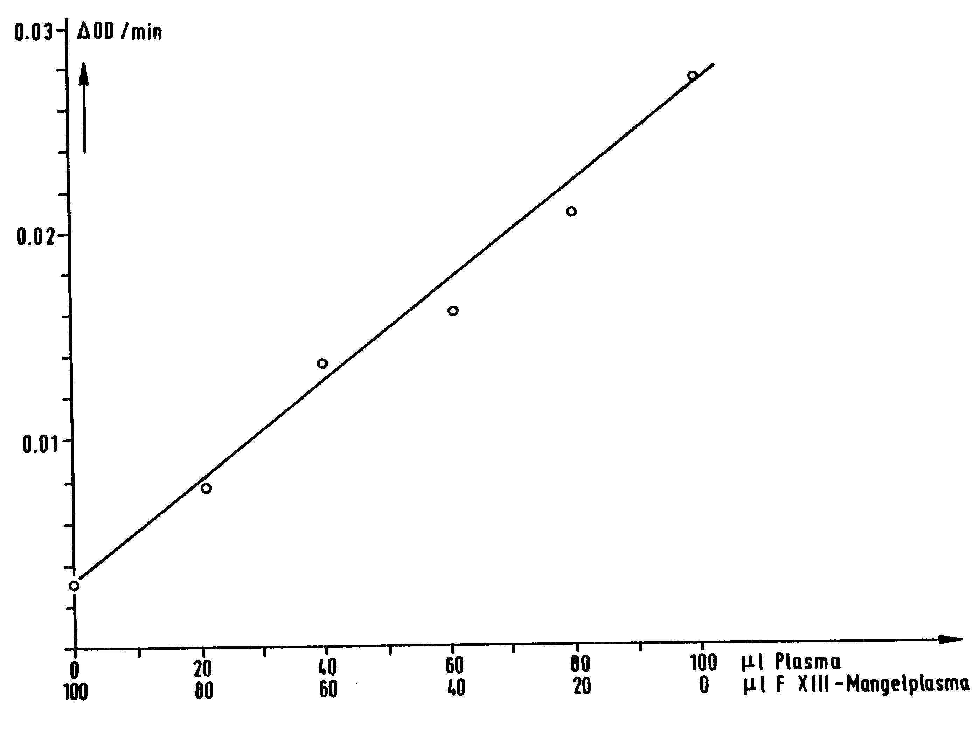

Fig. 1 zeigt die Geschwindigkeit der Veränderung der optischen Dichte (OD/Zeit) in Abhängigkeit vom Gehalt der Probe an F XIII. Unterschiedliche Konzentrationen an F XIII in den Proben wurden durch Mischen unterschiedlicher definierter Mengen eines Plasmas und eines F XIII Mangelplasmas erzeugt.

Gegenstand der Erfindung ist daher ein Kinetisches Verfahren zur Bestimmung von Faktor XIII in einer Faktor XIII enthaltenden, unbehandelten Probe, beispielweise Plasma, wobei
a) die Probe zunächst mit Thrombin in Gegenwart eines Fibrinaggregationshemmers, beispielweise Gly-Pro-Arg-Pro aktiviert wird;
b) die aktivierte Probe mit einem primären Amin, gegebenenfalls mit einer Puffersubstanz und einem glutaminhaltigen Peptidsubstrat für Faktor XIII versetzt wird; und
c) das gebildete Ammoniak mittels NADH, GLDH und Ketoglutarat photometrisch bestimmt wird.

Unbehandelte Proben, beispielsweise Plasma, werden direkt mit einer Thrombinlösung, die gegebenenfalls mit einer Pufferlösung zubereitet ist, voraktiviert und der dabei entstehende, aktivierte Faktor XIII mittels einer nachgeschalteten NADH-abhängigen Messung bestimmt. Durch einen Zusatz von Substanzen, die eine Aggregation von Fibrinmonomeren verhindern, beispielsweise Gly-Pro-Arg-Pro oder Gly-Pro-Arg-Pro-Arg, wird der störende Einfluß von Fibrinogen im Testansatz kompensiert. Vorzugsweise wird als Fibrinaggregationsinhibitor Gly-Pro-Arg-Pro benutzt.

Als Substrat für F XIII werden niedermolekulare glutaminhaltige Peptide, vorzugsweise Leu-Gly-Pro-Gly-Gln-Ser-Lys-Val-Ile-Gly-amid oder Leu-Gly-Pro-Gly-Gln-Ser-Lys-Val-Leu-Gly-amid verwendet.

Mit diesem Verfahren kann die Bestimmung von F XIII in einer F XIII-haltigen Lösung sicher und schnell durchgeführt werden. Die Testausführung beinhaltet vorzugsweise die folgenden Schritte:
- Vorlage der zu prüfenden Probe
- Zusatz des Fibrinaggregationhemmers, der gegebenenfalls in einer Pufferlösung gelöst vorliegt,
- Zusatz von Thrombin und Inkubation über einen Zeitraum von 3 - 10 min
- Messung der Ammoniakentwicklung, vorzugsweise durch das

System NADH, GLDH, Ketoglutarat, Amin und Substrat.

Diese Bestimmung benötigt nur ein kleines Probenvolumen. Vorzugsweise wurde die F XIII-Messung in einer 1 ml-Küvette bei 37°C ausgeführt, mit einem Probenvolumen von 25 bis 200 µl, besonders bevorzugt 50 µl.

Die Aktivierung des F XIII wird durch Zusatz von Thrombin in Gegenwart des Fibrinaggregationshemmers in einer Ca-Ionen enthaltenden Lösung ausgeführt. Zu diesem Zweck werden 2500 IE/l bis 20000 IE/l Thrombin, vorzugsweise 7000 IE/l in einem Puffer, der beispielsweise 10 mM bis 75 mM an HEPES, vorzugsweise 30 mM, und 75 mM bis 200 mM an NaCl, vorzugsweise 100 mM ist, gelöst. Ferner weist dieser Puffer eine Ca-Ionen-Konzentration von 20 mM bis 200 mM, vorzugsweise 60 mM, sowie eine Thiolkonzentration, vorzugweise Dithiothreitol-Konzentration von 2,5 mM bis 50 mM, vorzugsweise 17 mM, auf. Diesem Puffer wurde ein Fibrinaggregationshemmer, vorzugsweise Gly-Pro-Arg-Pro, in einer Konzentration von 0,5 mM bis 20 mM, vorzugsweise 1,5 mM zugesetzt und ein pH-Wert mit Salzsäure oder Natronlauge von 7,9 bis 8,2, vorzugsweise 7,6, eingestellt. Von diesem Puffer wurden 100 µl, vorzugsweise 150 µl, zu der Probe gegeben und 3 bis 10 Minuten, vorzugsweise 5 Minuten inkubiert.

Nach Ablauf der Aktivierungsphase des Faktors XIII werden die Nachweisreagenzien vorzugsweise in Form einer vorbereiteten Lösung zugegeben. Es wird auf jeweils 1 ml Gesamtlösung in der Küvette aufgestockt. Die vorbereitete Lösung der Nachweisreagenzien enthält alpha-Ketoglutarat, 1 mM bis 20 mM, vorzugsweise 7 mM sowie NADH, 0,05 mM bis 0,5 mM, vorzugsweise 0,125 mM sowie GLDH, 500 IU/l bis 6000 IU/l, vorzugsweise 300 IU/l sowie ein Substrat und ein Amin. Gegebenenfalls enthält diese Lösung noch eine Puffersubstanz, vorzugsweise Triethanolamin in einer Konzentration von 20 bis 200 mM, besonders bevorzugt 100 mM.

Als Substrate werden die glutaminhaltigen Peptide, wie bereits weiter oben beschrieben, eingesetzt, wobei die Endkonzentration an Peptid bei dem oben ausgeführten Testansatz in einem Bereich von 0,2 bis 5 mg, vorzugsweise 1 mg, pro ml Testansatz gewählt wird.

Als Amine haben sich primäre Amine als günstig erwiesen zu denen Substanzen wie Ethanolamin, Putrescin, Cadaverin, Diaminoethan, Aminoethan, bevorzugt jedoch Glycinethyl-oder -methylester zählen. Diese Amine werden bevorzugt zusammen mit dem Substrat in die vorbereitete Lösung der Nachweisreagenzien aufgenommen, wobei das Amin in einer Konzentration von 5 mM bis 75 mM, bevorzugt jedoch 25 mM, eingesetzt wird.

Der pH-Wert der Nachweisreagenzien-Lösung wird mit Salzsäure auf 7,2 bis 8,5, bevorzugt 8,0 eingestellt.

Die sich nun pro Zeiteinheit ändernde optische Dichte bei 340 ± 15 nm dient als direktes Maß für die F XIII-Aktivität.

Gegenstand der Erfindung ist auch eine Verpackung von Komponenten zur Verwendung in einem Verfahren zur Bestimmung von F XIII enthaltend einen Fibrinaggregationshemmer und ein glutaminhaltiges Peptid als Substrat für F XIII sowie gegebenenfalls andere für das Verfahren notwendige Reagenzien.

Die folgenden Beispiele erläutern dies näher:

### Beispiel 1

Bereitstellung der Lösung A:

Es wurden die folgenden Substanzen in 100 ml Wasser aufgelöst: 793 mg Hepes, 584 mg Natriumchlorid, 882 mg CaCl₂, 67 mg Gly-Pro-Arg-Pro, 257 mg Dithiothreitol und 666 IE Thrombin. Der pH-Wert wurde mit Natriumhydroxyd auf 7,6 eingestellt.

### Bereitstellung der Lösung N:

Es wurden die folgenden Substanzen in 100 ml Wasser gelöst: 10 mg NADH, 155 mg Dinatrium-alpha-Ketoglutarat, 213 Units GLDH, 375 mg Glycinethylesterhydrochlorid, 125 mg Leu-Gly-Pro-Gly-Gln-Ser-Lys-Val-Ile-Glyamid und 2,3 g Triethanolaminhydrochlorid. Der pH-Wert wurde mit Natriumhydroxyd auf 8,0 eingestellt.

### Testausführung:

50 µl Standard-Human-Plasma (1 Einheit F XIII/ml) wurden in eine 1 ml fassende Meßküvette (1 cm Lichtweg) pipettiert und bei 37°C mit 150 ml Lösung A inkubiert. Nach Ablauf von 5 Minuten wurden 800 µl Lösung N zugesetzt und die sich ändernde Extinktion bei 334 nm gemessen und mit einem Schreiber aufgezeichnet. Die Messung wurde für 5 Minuten verfolgt. Die optische Dichte zeigte eine Änderung von 0,015 pro Minute.

### Beispiel 2

Bereitstellung von Lösung A und N siehe Beispiel 1.

Es wurden 100 µl F XIII-Mangelplasma 5 Minuten mit 150 µl Lösung A bei 37°C inkubiert. Nach Zusatz von 750 µl Lösung N wurde die Aktivität bei 334 nm gemessen. Darauf folgend wurde 80 µl F XIII-Mangelplasma mit 20 µl Standard-Human-Plasma auf dieselbe Weise gemessen. Ebenso die Kombinationen 60 µl Mangelplasma/40 µl Standardplasma, 40 µl Mangelplasma/60 µl Standardplasma, 80 µl Standardplasma /20 µl Mangelplasma und 100 µl Standardplasma. Die gemessenen Werte sind aus dem Diagramm (Fig. 1) ersichtlich.

### Abkürzungen

- NADH: Nicotinamid-adenin-dinucleotid, reduziert
- GLDH: Glutamatdehydrogenase
- nm: Nanometer
- Gly: Glycin
- Pro: L-Prolin
- Arg: L-Arginin
- Leu: L-Leucin
- Gln: L-Glutamin
- Ser: L-Serin
- Lys: L-Lysin
- Val: L-Valin
- Ile: L-Isoleucin
- HEPES: 2-(4-(2-Hydroxyaethyl)-1-piperazino) ethansulfonsäure
- Ca: Calcium
- mM: millimol/Liter

## Patentansprüche

1. Kinetisches Verfahren zur Bestimmung von Faktor XIII in einer Faktor XIII enthaltenden, unbehandelten Probe, beispielweise Plasma, wobei
a) die Probe zunächst mit Thrombin in Gegenwart eines Fibrinaggregationshemmers, beispielweiseGly-Pro-Arg-Pro aktiviert wird;
b) die aktivierte Probe mit einem primären Amin, gegebenenfalls mit einer Puffersubstanz und einem glutaminhaltigen Peptidsubstrat für Faktor XIII versetzt wird; und
c) das gebildete Ammoniak mittels NADH, GLDH und Ketoglutarat photometrisch bestimmt wird.

2. Verfahren nach Anspruch 1, gekennzeichnet dadurch, daß 2500 IE/1 bis 20000 IE/1 Thrombin, vorzugsweise 7000 IE/1 gelöst in einem HEPES/Kochsalz-Puffer, der 10 mM bis 75 mM HEPES, vorzugsweise 30 mM, und 75 mM bis 200 mM NaCL, vorzugsweise 100 mM enthält, verwendet werden.

3. Verfahren nach Anspruch 1, gekennzeichnet dadurch, daß als Fibrinaggregationsinhibitor Gly-Pro-Arg-Pro benutzt wird.

4. Verfahren nach Anspruch 1, gekennzeichnet dadurch, daß als Substrat für F XIII ein niedermolekulares glutaminhaltiges Peptid, vorzugsweise Leu-Gly-Pro-Gly-Gln-Ser-Lys-Val-Ile-Gly-amid oder Leu-Gly-Pro-Gly-Gln-Ser-Lys-Val-Leu-Gly-amid verwendet wird.

5. Verfahren nach Anspruch 1, gekennzeichnet dadurch, daß 1mM bis 20 mM alpha-Ketoglutarat 0,05 mM bis 0,5 mM NADH sowie 500 IU/1bis 6000 IU/1 GLDH verwendet werden.

6. Verfahren nach Anspruch 1, gekennzeichnet dadurch, daß als Amin vorzugsweise Ethanolamin, Putrescin, Cadaverin, Diaminoethan oder Aminoethan, bevorzugt Glycinethyl oder methylester verwendet werden.

## Claims

1. A kinetic method for the determination of Factor XIII in a Factor XIII-containing untreated sample, for example plasma, where
a) the sample is first activated with thrombin in the presence of a fibrin aggregation inhibitor, for example Gly-Pro-Arg-Pro;
b) the activated sample is mixed with a primary amine, where appropriate with a buffer substance and a glutamine-containing peptide substrate for Factor XIII; and
c) the ammonia which is formed is determined photometrically using NADH, GLDH and ketoglutarate.

2. The method as claimed in claim 1, wherein 2500 IU/1 to 20,000 IU/1 thrombin, preferably 7,000 IU/1, are used dissolved in a HEPES/sodium chloride buffer which contains 10 mM to 75 mM HEPES, preferably 30 mM, and 75 mM to 200 mM NaCl, preferably 100 mM.

3. The method as claimed in claim 1, wherein Gly-Pro-Arg-Pro is used as fibrin aggregation inhibitor.

4. The method as claimed in claim 1, wherein a low molecular weight glutamine-containing peptide, preferably Leu-Gly-Pro-Gly-Gln-Ser-Lys-Val-Ile-Gly amide or Leu-Gly-Pro-Gly-Gln-Ser-Lys-Val-Leu-Gly amide, is used as substrate for Factor XIII.

5. The method as claimed in claim 1, wherein 1 mM to 20 mM alpha-ketoglutarate, 0.05 mM to 0.5 mM NADH and 500 IU/1 to 6,000 IU/1 GLDH are used.

6. The method as claimed in claim 1, wherein the amine which is preferably used is ethanolamine, putrescine, cadaverine, diaminoethane or aminoethane, preferably glycine ethyl or methyl ester.

## Revendications

1. Procédé cinétique pour la détermination du facteur XIII dans un échantillon non traité, par exemple du plasma, contenant du facteur XIII, dans lequel
a) on active d'abord l'échantillon avec de la thrombine en présence d'un inhibiteur d'agrégation de la fibrine, par exemple Gly-Pro-Arg-Pro;
b) on ajoute à l'échantillon activé une amine primaire, éventuellement avec une substance tampon et un substrat peptidique pour le facteur XIII, contenant de la glutamine; et
c) l'ammoniac formé est dosé photométriquement au moyen de NADH, GLDH et cétoglutarate.

2. Procédé selon la revendication 1, caractérisé en ce que l'on utilise 2 500 UI/1 à 20 000 UI/1, de préférence 7 000 UI/1 de thrombine en solution dans un tampon de chlorure de sodium/HEPES qui contient de 10 mM à 75 mM, de préférence 30 mM d'HEPES, et de 75 mM à 200 mM, de préférence 100 mM de NaCl.

3. Procédé selon la revendication 1, caractérisé en ce que l'on utilise comme inhibiteur d'agrégation de la fibrine Gly-Pro-Arg-Pro.

4. Procédé selon la revendication 1, caractérisé en ce que l'on utilise comme substrat pour F XIII un peptide à faible masse moléculaire contenant de la glutamine, de préférence Leu-Gly-Pro-Gly-Gln-Ser-Lys-Val-Ile-Gly-amide ou Leu-Gly-Pro-Gly-Gln-Ser-Lys-Val-Leu-Gly-amide.

5. Procédé selon la revendication 1, caractérisé en ce que l'on utilise de 1 mM à 20 mM d'α-cétoglutarate, de 0,05 à 0,5 mM de NADH ainsi que 500 UI/1 à 6 000 UI/1 de GLDH.

6. Procédé selon la revendication 1, caractérisé en ce que l'on utilise comme amine de préférence l'éthanolamine, la putrescine, la cadavérine, le diaminoéthane ou l'aminoéthane, en particulier l'ester méthylique ou éthylique de glycine.
